# EUROPEAN PATENT APPLICATION

(11) **EP 3 332 777 A1**
(43) Date of publication of application: **13.06.2018**
(21) Application number: 16832323.6
(22) Date of filing: 03.08.2016
(51) Int. Cl.: A61K 31/00, C07C 269/04, A61P 31/04

(54) **PHARMACEUTICAL COMPOSITION FOR INHIBITING DRUG-RESISTANT MICROORGANISM AND APPLICATION THEREOF**

(30) Priority: 03.08.2015 US 201562200516 P
(71) Applicant: Peptide Cham Biotech Co., Ltd., Kaohsiung City, Taiwan 801 (TW)
(72) Inventor: CHEN, Chiu-Hung, Kaohsiung City Taiwan 801 (TW); CHEN, Yeh, Nantou City Taiwan 540 (TW)
(74) Representative: Agasse, Stéphane
(86) International application number: PCT/CN2016/093065
(87) International publication number: WO 2017/020835

(57) **Abstract**

An antibiotic for inhibiting a drug-resistant microorganism. The antibiotic is Fmoc-Ala-OPfp, which is not toxic to human cells and can effectively inhibit a drug-resistant microorganism.

## Description

### FIELD

The disclosure relates to a pharmaceutical composition for inhibiting drug-resistant microorganisms and use thereof.

### BACKGROUND

Drug resistance refers to the reduction in effectiveness of a medication for treating diseases or ameliorating the patient's symptoms. When the concentration of a drug to be administered is not sufficient to kill or inhibit pathogens, the residual pathogens might have resistance to the administered drug. For example, bacteria might have drug resistance due to DNA mutations induced by reactive oxygen species that are generated by antibiotics.

The antibiotic resistance mechanism of bacteria is basically controlled by genes which are collectively referred to as drug resistance genes. Among these drug resistance genes, some are inherent genes, and some may be obtained from other bacteria through the transmission of plasmids or transposons. Antibiotics certainly eliminate the bacteria that can be killed by the same, but those with drug resistant genes survive therefrom. Therefore, antibiotics result in more and more antibiotic-resistant bacteria. This is the reason why the more frequently antibiotics are used, the higher the chance that antibiotic-resistant bacteria are found will be.

Recently, due to lack of drugs for effectively treating the infection by drug-resistant bacteria and due to excessive abuse of antibiotics, mutant bacteria having strong drug resistance are formed and result in more than 40,000 deaths each year in Europe and America. Therefore, all the countries are developing new antibiotics to kill these drug-resistant bacteria, also known as "superbugs".

Ampicillin, serves as a first-line antibiotic in the clinical field, is commonly applied by medical institutes and livestock industry. Due to abuse of this antibiotic, more and more bacteria having drug resistance are generated. *E. coli* resistant to ampicillin has been found in 71% of outpatients in Taiwan and 39.3% of outpatients in the United States. Such phenomenon is observed not only on the first-line antibiotics, but also on vancomycin, which is used when other antibiotics are not therapeutically effective, and the abuse of which also leads to bacteria having resistance, e.g. vancomycin-resistant *Enterococcus* (VRE).

For saving cost, pharmaceutical companies have been pursuing simpler drug development methods by only modifying the structure of current antibiotics, such as replacement of the functional groups thereof. However, after such modified antibiotics are utilized for a certain period of time, drug-resistant bacteria still develop new resistance thereagainst. If design of new antibiotics is not feasible, there will be no drugs potent against bacteria, causing the patients that have been diagnosed as bacterial infection to be incurable.

### SUMMARY

In view of the above, the present disclosure discloses an antibiotic which may inhibit a microorganism with drug resistance. Such antibiotic is Fmoc-Ala-OPfp.

Moreover, the present disclosure provides a pharmaceutical composition for inhibiting the growth of a microorganism. The pharmaceutical composition is an antibiotic, i.e. N-(9-fluorenylmethoxycarbonyl)-L-alanine pentafluorophenyl (Fmoc-Ala-OPfp, CAS: 86060-86-8), which is an alanine (Ala) derivative having a structure of formula I:

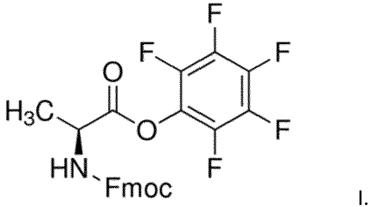

Preferably, the aforementioned microorganism with drug resistance is selected from one or more of Gram-positive bacteria, *Staphylococcus aureus, Staphylococcus epidermidis, Streptococcus pneumoniae, Enterococci, Clostridium tetani* and *Clostridium botulinum.*

Preferably, the aforementioned microorganism with drug resistance is *Staphylococcus aureus.*

Preferably, an effective concentration of the aforementioned pharmaceutical composition (i.e. the antibiotic) ranges from 2.5 µM to 100 µM.

Preferably, the effective concentration of the aforementioned pharmaceutical composition (i.e. the antibiotic) is 2.5 µM.

Preferably, the aforementioned drug resistance may be a tolerance to vancomycin.

The present disclosure further provides a pharmaceutical composition capable of inhibiting the growth of microorganisms. Such pharmaceutical composition includes Fmoc-Ala-OPfp.

Still further, the present disclosure provides a pharmaceutical composition for use in inhibiting the growth of microorganisms. Such pharmaceutical composition includes Fmoc-Ala-OPfp.

### BRIEF DESCRIPTION OF THE DRAWINGS

Figure 1 shows the hemolysis test result of a pharmaceutical composition (Fmoc-Ala-OPfp) of the present disclosure.
Figure 2 shows the cytotoxicity test result of the pharmaceutical composition (Fmoc-Ala-OPfp) of the present disclosure on human cells.
Figure 3 is a comparative diagram showing the inhibiting effects of the pharmaceutical composition (Fmoc-Ala-OPfp) of the present disclosure as well as broad-spectrum antibiotics, ampicillin and vancomycin, on drug-resistant *Staphylococcus aureus.*

### DETAILED DESCRIPTION

### Example 1. Antimicrobial activity test

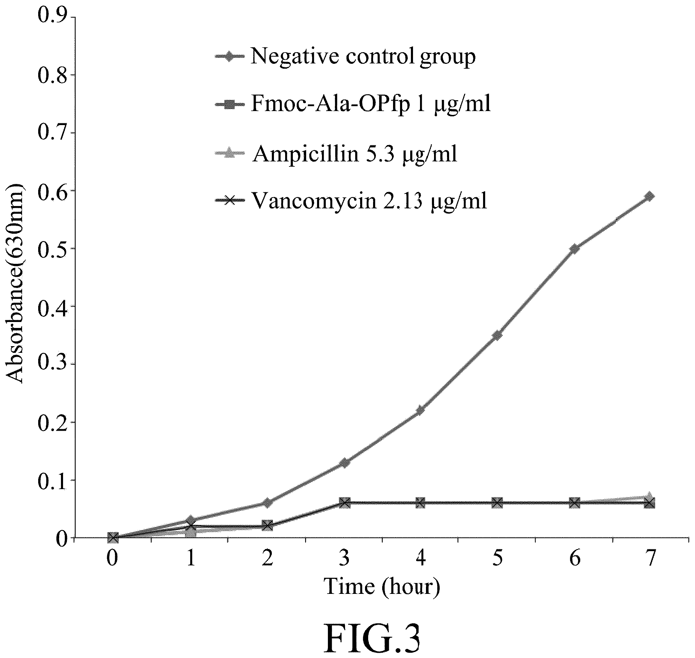

Referringto Figure 3, bacteria were cultured for the test in this example. Culture mediums were respectively added with the pharmaceutical composition of the present disclosure (Fmoc-Ala-Opfp), ampicillin and vancomycin at a specific concentration to serve as experimental groups, and a culture medium without any drugs added therein served as a control group. An equal amount of *Staphylococcus aureus* was inoculated in each of the culture mediums, followed by culturing in an incubator at 37°C. The concentrations of the cultured bacteria (OD₆₃₀) at different time points were determined to investigate the bacterial inhibiting effects of Fmoc-Ala-OPfp, ampicillin and vancomycin.

Even after 8 hours of the bacterial inoculation, the small amount (1 µg/mL) of Fmoc-Ala-OPfp added in the culture medium could still exhibit a bacterial inhibiting effect equivalent to those provided by 5.3 µg/mL of ampicillin and 5.3 µg/mL of vancomycin, indicating that Fmoc-Ala-OPfp could effectively inhibit the bacterial growth.

### Example 2. Hemolysis test

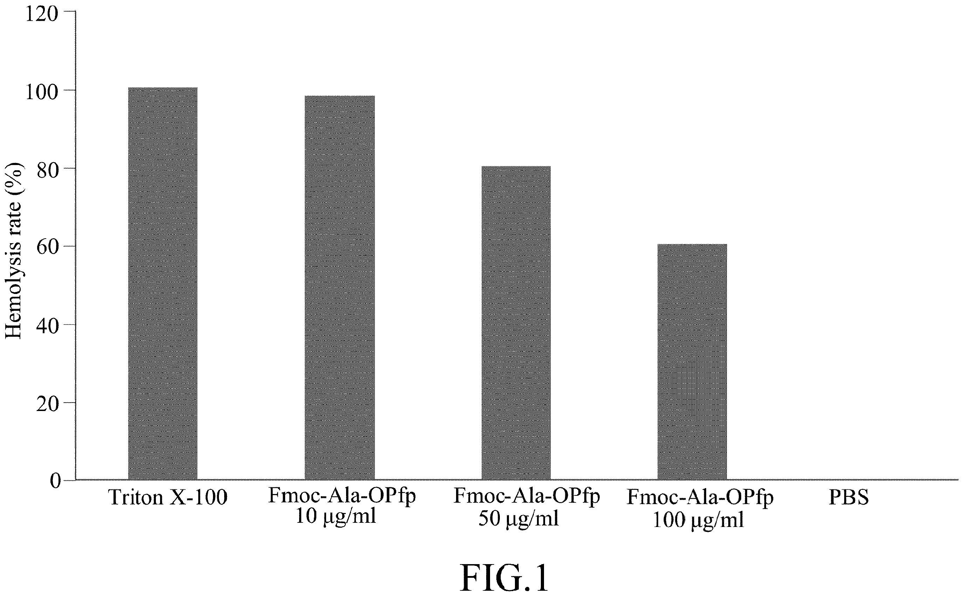

Referring to Figure 1, fresh human red blood cells were washed with 0.9% physiological saline for three times, and then were resuspended with the physiological saline having a concentration of 2% (V/V). 50 µL of the resulting red blood cell suspension was added into a 96-well plate, followed by adding a respective one of the following: 50 µL of the pharmaceutical composition of this disclosure (10, 50 or 100 µg/mL of the antibiotic Fmoc-Ala-OPfp) and 50 µL of Triton X-100. The final concentration of the red blood cells was 2% (V/V). The test sample was incubated in an incubator with slight shaking at 37°C for 1 hour, and then was centrifuged under 2000 g for 30 minutes. 50 µL of the sample supernatant was added into a new 96-well plate to determine the absorbance thereof at 490 nm (OD₄₉₀). The physiological saline (i.e. phosphate buffered solution, also known as PBS) and Triton X-100 respectively served as a negative control group and a positive control group.

### Example 3. Cell Culture

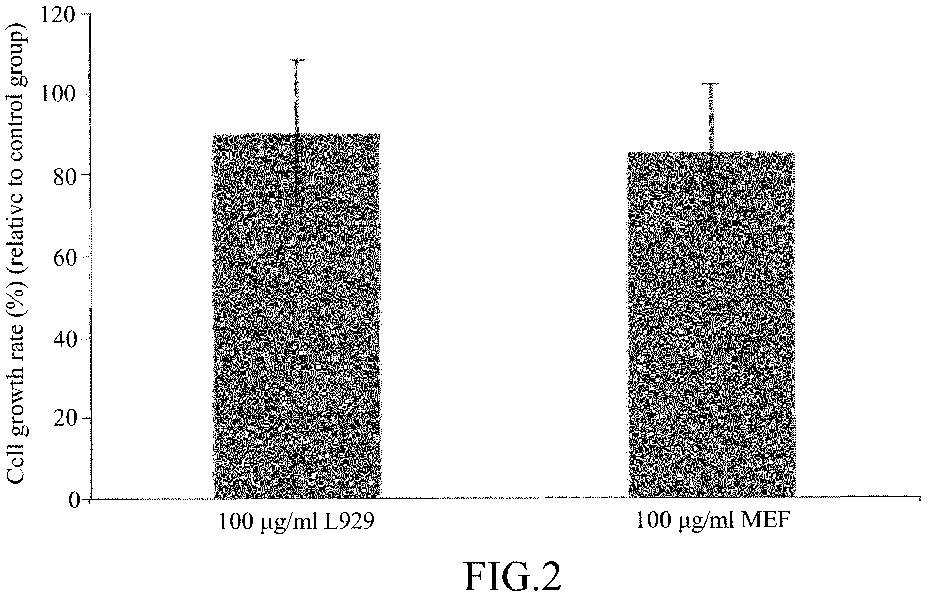

Referring to Figure 2, L292 and MEF cell lines, i.e. mammalian cell lines, were respectively added into Dulbecco's modified Eagle mediums (DMEM) supplemented with 100 µg/mL streptomycin, 100 U/mL penicillin and 10% (v/v) fetal bovine serum, followed by cultivation in a humidified incubator containing 5% CO₂ and having a temperature of 37°C.

### Example 4. Analysis of survival rate

An MTT assay was conducted for determining the cytotoxicity of the test compound against mammalian cells. The cells were cultured in a sterilized 96-well plate with 100 µL of a culture medium for 24 hours, and then the test compound was added into the culture medium followed by cultivation for 24 hours. Each well of the 96-well plate was added with a solution of 3-(4,5- dimethylthiazol-2-yl)-2,5-diphenyltetrazolium bromide (MTT), followed by incubation for 4 hours. The liquid portion was removed, and 100 µL of dimethyl sulfoxide (DMSO) was added for dissolving the precipitate. The absorbance at 570 nm was measured by an enzyme immunoassay analyzer.

As shown in Figures 2 and 3, concerning the hemolysis test on human red blood cells and the toxicity test of Fmoc-Ala-OPfp by the MTT assay, Fmoc-Ala-OPfp, at a concentration of 100 µg/mL, still exhibited low hemolytic activity, and 100 µg/mL of Fmoc-Ala-OPfp only showed 10-20% of toxicity against cells in the MTT assay.

The detailed description above serves to illustrate the practicable embodiment(s) of the present disclosure. However, such embodiment(s) should not be utilized to limit the claimed scope of the present disclosure. Accordingly, the claimed scope of the present disclosure is intended to encompass all equivalent practice or changes without departing from the spirit of the invention.

## Claims

1. A pharmaceutical composition for inhibiting a microorganism, **characterized by** comprising N-(9-fluorenylmethoxycarbonyl)-L-alanine pentafluorophenyl (Fmoc-Ala-OPfp), N-(9-fluorenylmethoxycarbonyl)-L-alanine pentafluorophenyl having a structure of formula I:

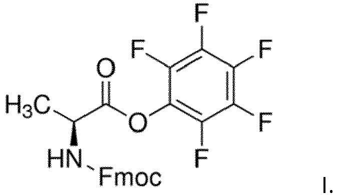

2. The pharmaceutical composition of claim 1, **characterized in that** the microorganism is a microorganism with drug-resistance.

3. The pharmaceutical composition of claim 2, **characterized in that** the microorganism with drug-resistance is one or more selected from the group consisting of Gram-positive bacteria, *Staphylococcus aureus, Staphylococcus epidermidis, Streptococcus pneumoniae, Enterococci, Clostridium tetani* and *Clostridium botulinum.*

4. The pharmaceutical composition of claim 2, **characterized in that** the microorganism with drug-resistance is *Staphylococcus aureus.*

5. The pharmaceutical composition of claim 1, **characterized in that** an effective concentration of N-(9-fluorenylmethoxycarbonyl)-L-alanine pentafluorophenyl is 2.5-100 µM.

6. The pharmaceutical composition of claim 1, **characterized in that** an effective concentration of N-(9-fluorenylmethoxycarbonyl)-L-alanine pentafluorophenyl is 2.5 µM.

7. The pharmaceutical composition of claim 2, **characterized in that** the drug resistance is a tolerance to vancomycin.

8. Use of a pharmaceutical composition for inhibiting a microorganism, **characterized by** the pharmaceutical composition comprising Fmoc-Ala-OPfp.

9. The pharmaceutical composition of claim 8, **characterized in that** the microorganism is a drug-resistant microorganism.
